# EUROPÄISCHE PATENTSCHRIFT

(11) **EP 0 811 616 B1**
(45) Veröffentlichungstag und Bekanntmachung des Hinweises auf die Patenterteilung: **04.05.2005**
(21) Anmeldenummer: 97108569.1
(22) Anmeldetag: 28.05.1997
(51) Int. Cl.: C07D 233/78, A61K 31/415

(54) **Salze des 3-(2-(4-(4-(Amino-imino-methyl)-phenyl)-4-methyl-2,5-dioxo-imidazolidin-1-yl)-acetylamino)-3-phenyl-propionsäure-ethylesters**
Salts of 3-(2-(4-(4-(Amino-imino-methyl)-phenyl)-4-methyl-2,5-dioxo-imidazolidin-1-yl)-acetylamino)-3-phenyl-propionic acid ethylester
Sels de l'ester éthylique de l'acide 3-(2-(4-(4-(Amino-imino-méthyl)-phényl)-4-méthyl-2,5-dioxo-imidazolidin-1-yl)-acétylamino)-3-phényl-propionique

(30) Priorität: 05.06.1996 DE 19622489
(43) Veröffentlichungstag der Anmeldung: 10.12.1997
(73) Patentinhaber: Aventis Pharma Deutschland GmbH, 65929 Frankfurt am Main (DE)
(72) Erfinder: Stilz, Hans Ulrich, Dr., 65929 Frankfurt (DE); Beck, Gerhard, Dr., 60320 Frankfurt (DE); Radau, Manfred, Dr., 65779 Kelkheim (DE)

(56) Entgegenhaltungen:
- WO-A-95/14008
- WO-A-96/33976

## Beschreibung

Die vorliegende Erfindung betrifft das (S)-3-(2-((S)-4-(4-(Amino-imino-methyl)-phenyl)-4-methyl-2,5-dioxo-imidazolidin-1-yl)-acetylamino)-3-phenyl-propionsäureethylester-Hydrogenmaleat-Salz der Formel I, 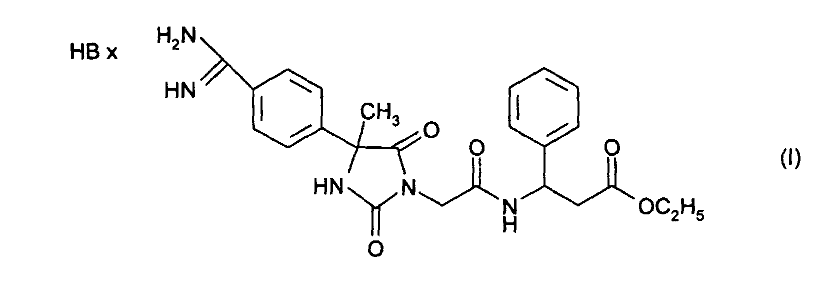 worin HB Maleinsäure bedeutet und am chiralen Zentrum im lmidazolidinring die (S)-Konfiguration und am chiralen Zentrum in der Propionsäureeinheit die (S)-Konfiguration vorliegt, sowie seine physiologisch verträglichen Salze, Verfahren zu seiner Herstellung und seine Verwendung in Arzneimitteln.

3-(2-(4-(4-(Amino-imino-methyl)-phenyl)-4-methyl-2,5-dioxo-imidazolidin-1-yl)-acetylamino)-3-phenyl-propionsäure-ethylester-hydrochloride und ihre pharmakologischen Eigenschaften sind bereits in der PCT-Anmeldung PCT/EP94/03491 (WO-A-95/14008) beschrieben. Die Hydrochlorid-Salze des (S)-3-(2-((S)-4-(4-(Amino-imino-methyl)-phenyl)-4-methyl-2,5-dioxo-imidazolidin-1-yl)-acetylamino)-3-phenyl-propionsäure-ethylesters, des (R)-3-(2-((R)-4-(4-(Aminoimino-methyl)-phenyl)-4-methyl-2,5-dioxo-imidazolidin-1-yl)-acetylamino)-3-phenylpropionsäure-ethylesters, des (S)-3-(2-((R)-4-(4-(Amino-imino-methyl)-phenyl)-4-methyl-2,5-dioxo-imidazolidin-1-yl)-acetylamino)-3-phenyl-propionsäure-ethylesters und des (R)-3-(2-((S)-4-(4-(Amino-imino-methyl)-phenyl)-4-methyl-2,5-dioxoimidazolidin-1-yl)-acetylamino)-3-phenyl-propionsäure-ethylesters haben aber den Nachteil, daß sie amorph sind und nicht kristallin erhalten werden können. Die obigen Hydrochlorid-Salze können daher nicht durch Kristallisation gereinigt werden und sind somit kaum brauchbar für den Einsatz als Wirkstoff in Arzneimitteln, für die vom Gesetzgeber genau definierte Reinheitsgrade der Inhaltsstoffe vorgeschrieben sind, und als Zielprodukt bei der technischen Synthese der pharmakologisch aktiven Substanz, bei dessen Isolierungs- und Reinigungverfahren ebenfalls nach den gesetzlichen Vorgaben genau definierte Bedingungen eingehalten werden müssen. Auch sind die amorphen Hydrochlorid-Salze aufgrund ihrer physikalischen Eigenschaften und ihrer Handhabbarkeit für die galenische Herstellung von pharmazeutischen Zubereitungen wie z. B. Tabletten nur wenig geeignet.

Aufgabe vorliegender Erfindung ist es, den (S)-3-(2-((S)-4-(4-(Amino-imino-methyl)-phenyl)-4-methyl-2,5-dioxo-imidazolidin-1-yl)-acetylamino)-3-phenyl-propionsäure-ethylester in einer geeigneten, nicht hygroskopischen Form bereitzustellen, die auf einfache Art die Einhaltung der geforderten Reinheitsgrade ermöglicht und die Anforderungen bei der technischen Synthese und die galenischen Anforderungen erfüllt.

Diese Aufgabe wird in überraschender Weise gelöst durch die Bereitstellung des Maleinsäuresalzes des (S)-3-(2-((S)-4-(4-(Amino-imino-methyl)-phenyl)-4-methyl-2,5-dioxo-imidazolidin-1-yl)-acetylamino)-3-phenyl-propionsäure-ethylesters, das diesen Ethylester und Maleinsäure in Salzform (d. h. in Form eines Säureadditionssalzes) im molaren Verhältnis von ca. 1:1 enthält.

Gegenstand der vorliegenden Erfindung ist somit das 3-(2-(4-(4-(Amino-imino-methyl)-phenyl)-4-methyl-2,5-dioxo-imidazolidin-1-yl)-acetylamino)-3-phenyl-propionsäure-ethylester-Salz der Formel I, 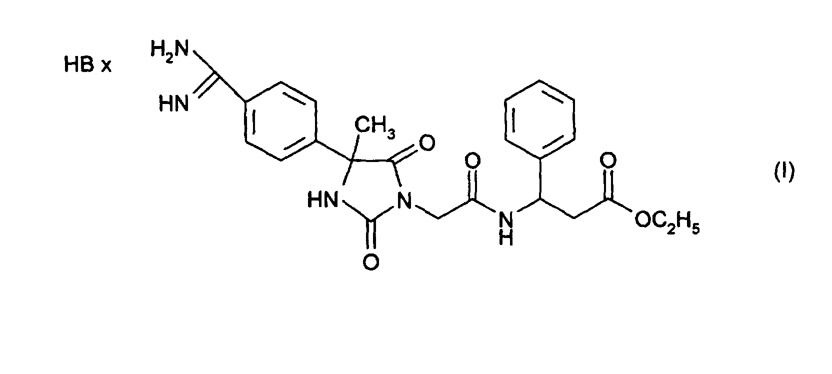 worin HB Maleinsäure bedeutet und am chiralen Zentrum im Imidazolidinring die (S)-Konfiguration und am chiralen Zentrum in der Propionsäureeinheit die (S)-Konfiguration vorliegt, sowie seine physiologisch verträglichen Salze.

Die Verbindung der Formel I ist kristallin und nicht hygroskopisch und besitzt somit nicht vorhersehbare Vorteile. Sie kann unter definierten Bedingungen kristallisiert und durch Umkristallisation gereinigt werden und eignet sich für den Einsatz in Arzneimitteln und die galenische Herstellung von Arzneiformen.

Die vorliegende Erfindung betrifft das Stereoisomer mit (S)-Konfiguration am chiralen Zentrum im Imidazolidinring und (S)-Konfiguration am chiralen Zentrum in der Propionsäureeinheit, also das (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Salz der Formel Ia, 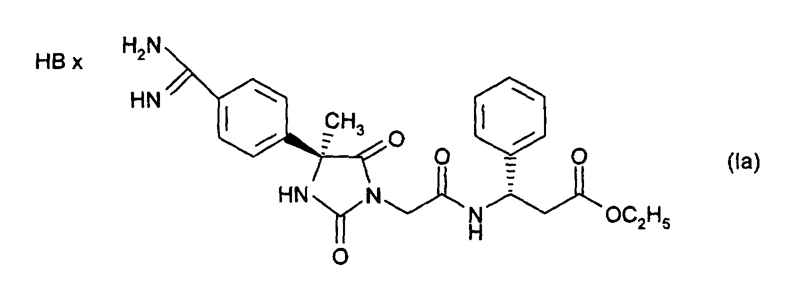 in der HB Maleinsäure bedeutet, sowie dessen physiologisch verträgliche Salze.

In der erfindungsgemäßen Verbindung der Formel I, die (S)-3-(2-((S)-4-(4-(Amino-imino-methyl)-phenyl)-4-methyl-2,5-dioxo-imidazolidin-1-yl)-acetylamino)-3-phenyl-propionsäure-ethylester und Maleinsäure im molaren Verhältnis von ca. 1:1 enthält, ist eine der beiden COOH-Gruppen der Maleinsäure durch die basische Amidinogruppe neutralisiert und liegt in der Salzform, d. h. als negativ geladene Carboxylatgruppe, vor (die Amidinogruppe im Ethylester liegt nach der Protonierung durch die Maleinsäure dann als positiv geladene Amidiniumgruppe vor). Die zweite der beiden COOH-Gruppen der Maleinsäure kann in der erfindungsgemäßen Verbindung in der Säureform vorliegen, d. h. als COOH-Gruppe, oder sie kann in der Salzform vorliegen, d. h. als Carboxylatgruppe. Gegenstand der vorliegenden Erfindung sind sowohl das Hydrogenmaleat, in dem noch eine COOH-Gruppe vorliegt und das bevorzugt ist, als auch die davon abgeleiteten physiologisch verträglichen Salze, die mit anorganischen oder organischen Basen daraus gebildet werden.

Physiologisch verträgliche Salze sind dabei insbesondere pharmazeutisch verwendbare oder nicht-toxische Salze. Kationen, die in solchen Salzen vorliegen können, leiten sich z. B. von Alkali- und Erdalkalimetallen wie Natrium, Kalium, Magnesium oder Calcium, vom Ammoniak oder von physiologisch verträglichen organischen Aminen wie Triethylamin, Ethanolamin oder Tris-(2-hydroxy-ethyl)-amin, ab. Die Salze der Verbindung der Formel I können direkt bei dem im folgenden beschriebenen Herstellverfahren erhalten werden, indem darin z. B. geeignete Salze der Maleinsäure eingesetzt oder geeignete Basen zugesetzt werden. Die Salze können aber auch erhalten werden, indem zunächst hergestelltes Hydrogenmaleat der Formel I, in dem noch eine COOH-Gruppe vorliegt, mit geeigneten Basen behandelt wird, z. B. Alkali- oder Erdalkalihydroxiden, -carbonaten oder -hydrogencarbonaten, Erdalkalioxiden oder Aminen. Dabei kann die COOH-Gruppe vollständig in die Salzform überführt werden oder nur teilweise. Das Ausmaß, in dem eine solche Salzbildung durchgeführt wird, hängt z. B. vom pH-Wert ab, der bei der vorgesehenen Verwendung angestrebt wird. Die Überführung der freien COOH-Gruppe - wiederum vollständig oder nur teilweise - in die Salzform kann auch erst erfolgen, wenn das Hydrogenmaleat der Formel I zusammen mit basischen Substanzen bei der Herstellung pharmazeutischer Zubereitungen eingesetzt wird.

Die Verbindung der Formel I kann dadurch hergestellt werden, daß mit Verbindungen der allgemeinen Formel II, 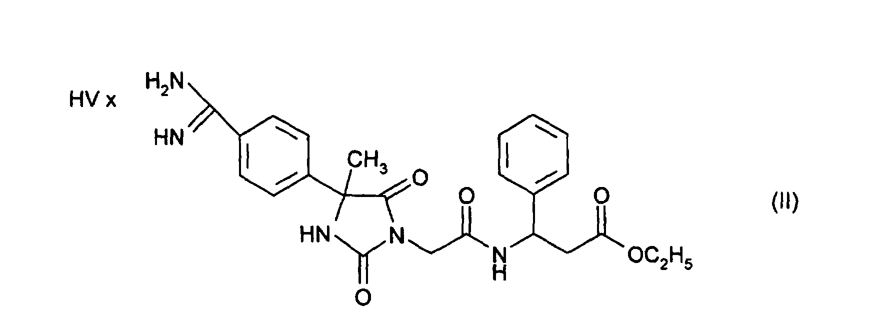 in der HV für eine beliebige anorganische oder organische, von Maleinsäure verschiedene Säure steht und am chiralen Zentrum im Imidazolidinring die (S)-Konfiguration und am chiralen Zentrum in der Propionsäureeinheit die (S)-Konfiguration vorliegt, nach üblichen, dem Fachmann bekannten Methoden ein Anionenaustausch mit Maleinsäure und/oder Maleaten durchgeführt wird. Beispiele für Säuren der allgemeinen Formel HV sind Chlorwasserstoff, Bromwasserstoff, Schwefelsäure, Phosphorsäure, Essigsäure, Propionsäure oder Methansulfonsäure. Beispiele für Verbindungen der allgemeinen Formel II sind die Verbindungen der Formeln IIa und IIb, 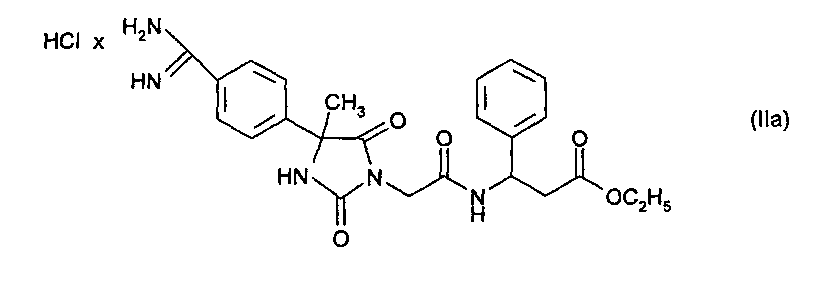 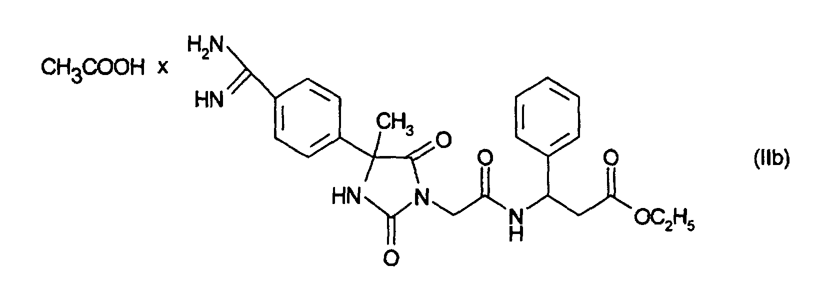 in denen HV in der allgemeinen Formel II für Chlorwasserstoff bzw. Essigsäure steht, und in denen am chiralen Zentrum im lmidazolidinring die (S)-Konfiguration und am chiralen Zentrum in der Propionsäureeinheit die (S)-Konfiguration vorliegt. Die Ausgangsverbindungen der allgemeinen Formel II können, wie in den PCT-Anmeldungen PCT/EP94/03491 und PCT/EP96/01572 (WO-A-96/33976) beispielsweise für das Hydrochlorid (Salz mit Chlorwasserstoff) oder für das Salz mit Essigsäure beschrieben, hergestellt werden. Als Maleate können beim Anionenaustausch beispielsweise Natrium-, Lithium-, Kalium- und Ammonium-Salze der Maleinsäure oder Salze der Maleinsäure mit organischen Ammoniumkationen eingesetzt werden. Bevorzugt wird Maleinsäure eingesetzt.

Für den Anionenaustausch kann beispielsweise eine Lösung von Verbindungen der allgemeinen Formel II über einen mit Maleinsäure beladenen Ionenaustauscher chromatographiert werden. Geeignete Lösungsmittel hierfür sind beispielsweise Wasser, Alkohole wie Methanol, Ethanol, Butanol oder i-Propanol, sowie Gemische dieser Lösungsmittel, z. B. Wasser-Alkohol-Gemische. Es können handelsübliche Anionenaustauschermaterialien eingesetzt werden, die nach der üblichen Vorgehensweise zunächst in die Maleinsäure-Form überführt werden bzw. bei wiederholter Verwendung zu dieser Form regeneriert werden. Der Anionenaustausch wird normalerweise bei Temperaturen von -10 bis 40 °C, insbesondere -5 bis 30 °C, vorzugsweise 0 bis 25 °C, durchgeführt. Aus dem Eluat der Ionenaustauscherchromatographie kann das gewünschte Produkt dann - gewünschtenfalls nach Einengen - z. B. durch Abkühlen und/oder Ausfällen auskristallisiert werden und durch Filtrieren oder Zentrifugieren isoliert werden.

Für den Anionenaustausch können beispielsweise auch Verbindungen der allgemeinen Formel II in einem Lösungsmittel mit Maleinsäure und/oder Maleaten vereinigt werden. Unter Lösungsmittel sind dabei auch Gemische von zwei oder mehr Lösungsmitteln zu verstehen. Geeignete Lösungsmittel sind beispielsweise Wasser, Alkohole, beispielsweise solche mit 1 bis 8 C-Atomen, insbesondere solche mit 1 bis 6 C-Atomen, vorzugsweise solche mit 1 bis 4 C-Atomen, Ether wie Dioxan, Tetrahydrofuran, Ethylenglykol- und Diethylenglykolmono- und -dimethylether, Ketone wie Aceton oder Butanon, Nitrile wie Acetonitril, Dimethylformamid, Dimethylsulfoxid, sowie Gemische solcher Lösungsmittel, z. B. Gemische aus Wasser und Alkoholen. Als Alkohole können z. B. eingesetzt werden Methanol, Ethanol, i- und n-Propanol, n-, i-, sec-, und tert.-Butanol, n-, i-, sec- und tert.-Pentanol, n-Hexanol, 2-Ethylbutanol, 2-Ethylhexanol, Isooctylalkohol, Cyclopentanol, Methylcyclohexanol (Gemisch) oder Benzylalkohol.

Das molare Verhältnis Verbindung der allgemeinen Formel II : Maleinsäure bzw. Maleat beträgt bei dieser Vorgehensweise normalerweise 1:1 bis 1:10, bevorzugt 1:1 bis 1:2, besonders bevorzugt ca. 1:1. Es können die Verbindungen der allgemeinen Formel II vorgelegt werden und die Maleinsäure bzw. die Maleate oder Gemische aus Maleinsäure und Maleaten zugesetzt werden oder umgekehrt, und es können auch beide Komponenten gleichzeitig in das Reaktionsgefäß dosiert werden. Die Komponenten können in Form von Lösungen vereinigt werden, je nach der Ausführungsart des Anionenaustausches kann es aber auch günstig sein, Suspensionen vorzulegen und/oder Suspensionen oder Feststoffe zuzusetzen. Der Anionenaustausch wird normalerweise bei Temperaturen von -10 bis 40 °C, insbesondere -5 bis 30 °C, vorzugsweise 0 bis 25 °C, durchgeführt. Zur Isolierung kann das erhaltene Maleinsäuresalz - gewünschtenfalls nach Einengen - z. B. durch Abkühlen und/oder Ausfällen auskristallisiert werden und durch Filtrieren oder Zentrifugieren abgetrennt werden. Je nach den Anforderungen kann es dann noch gewaschen und gewünschtenfalls z. B. durch Umkristallisieren oder Digerieren weiter gereinigt werden.

In einem bevorzugten Verfahren zur Herstellung des erfindungsgemäßen Maleinsäuresalzes der Formel I wird das Essigsäuresalz der Formel IIb als Ausgangsverbindung eingesetzt. Besonders bevorzugt ist es dabei, dieses Salz in einem Lösungsmittel mit Maleinsäure zu vereinigen.

Die in den Anionenaustausch eingesetzten Verbindungen der allgemeinen Formel II können in einer bevorzugten Vorgehensweise auf folgendem Weg erhalten werden. Die Verbindung der Formel III, 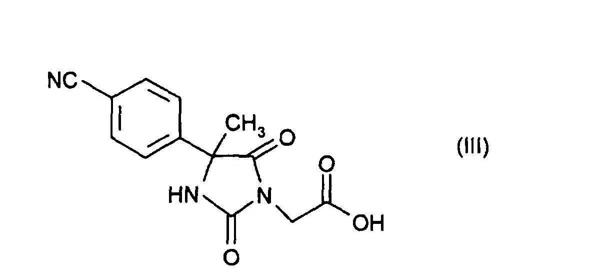 deren Herstellung gemäß den Angaben in den PCT-Anmeldungen PCT/EP94/03491 und PCT/EP96/01572 erfolgen kann, kann in Gegenwart wasserbindender Agentien, wie z. B. Dicyclohexylcarbodiimid (DCC), O-((Cyan(ethoxycarbonyl)-methylen)amino)-N,N,N',N'-tetramethyluronium-tetrafluoroborat (TOTU) oder Propylphosphonsäureanhydrid (PPA), unter den üblichen Bedingungen für solche Reaktionen mit der Verbindung der Formel IV, 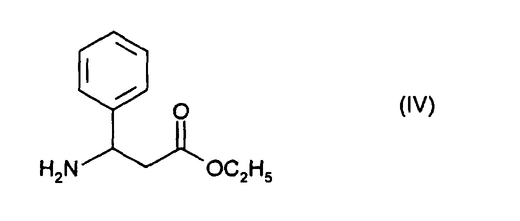 deren Herstellung nach in der Literatur beschrieben Verfahren erfolgen kann (siehe z. B. E. Juaristi, D. Quintana, J. Escalante, Aldrichimica Acta, Vol. 27, No.1, 1994, 3-11; D.C. Cole, Tetrahedron, Vol. 50, 1994, 9517-9582), zur Verbindung der Formel V 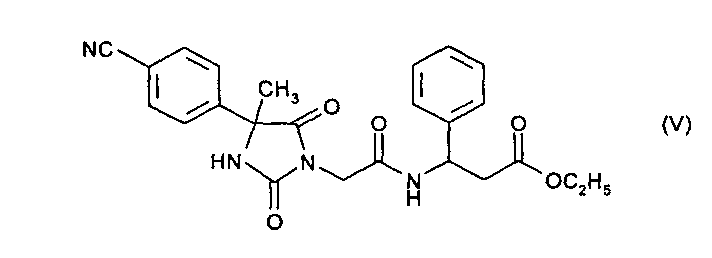 gekuppelt werden. In den Verbindungen der Formeln III, IV und V liegt am chiralen Zentrum im Imidazolidinring die (S)-Konfiguration und am chiralen Zentrum in der Propionsäureeinheit die (S)-Konfiguration vor.

Die Verbindung der Formel V kann z. B. gemäß den üblichen Verfahren zur Überführung von Nitrilen in Amidoxime in einem geeigneten Lösungsmittel, z. B. in einem Alkohol wie Methanol oder Ethanol, mit Hydroxylamin bzw. mit einem Hydroxylammoniumsalz und einer Base, z. B. einem tertiären Amin oder einem Alkalimetallhydroxid, -carbonat oder -hydrogencarbonat, in die Verbindung der Formel VI, 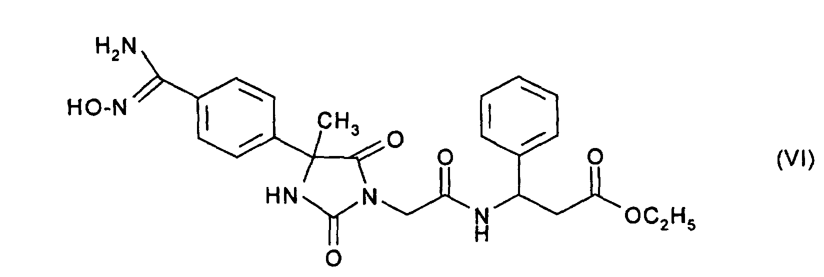 in der am chiralen Zentrum im Imidazolidinring die (S)-Konfiguration und am chiralen Zentrum in der Propionsäureeinheit die (S)-Konfiguration vorliegt, überführt werden, die dann durch Reduktion unter üblichen Bedingungen, z. B. durch Hydrierung mit Wasserstoff in Gegenwart von Metallkatalysatoren, und durch Zusatz der Säure der allgemeinen Formel HV, insbesondere durch Hydrierung in Gegenwart der Säure der allgemeinen Formel HV, in die Verbindungen der allgemeinen Formel II überführt werden können. Als Metallkatalysatoren bei einer Hydrierung, die unter Atmosphärendruck oder bevorzugt bei erhöhtem Druck durchgeführt werden kann, können z. B. Edelmetallkatalysatoren wie Palladium auf Kohle verwendet werden. Zur bevorzugten Herstellung der Verbindungen der Formel IIb kann dabei z. B. in einem geeigneten Lösungsmittel in Gegenwart von Essigsäure oder auch in Essigsäure als Lösungsmittel hydriert werden.

Die pharmakologischen Eigenschaften der Verbindung der Formel I sind von der Tatsache, daß es sich um ein Maleinsäuresalz handelt, unabhängig. Wie z. B. das entsprechende Hydrochlorid hat die erfindungsgemäße Verbindung der Formel I die Fähigkeit, die Zell-Zell-Adhäsion zu hemmen, die auf der Interaktion von Arg-Gly-Asp-enthaltenden Proteinen, wie Fibronectin, Fibrinogen oder des von Willebrand-Faktors, mit den sogenannten Integrinen beruhen. Integrine sind Transmembran-Glykoproteine, Rezeptoren für Arg-Gly-Asp-enthaltende Zellmatrix-Proteine (E. Ruoslahti und M.D. Pierschbacher, Science 238 (1987) 491-497; D.R. Phillips, I.F. Charo, L.V. Parise und L.A. Fitzgerald, Blood 71 (1988) 831-843). Außerdem hemmt sie die Bindung weiterer adhäsiver Proteine, wie Vitronectin, Kollagen und Laminin an die entsprechenden Rezeptoren auf der Oberfläche verschiedener Zelltypen. Insbesondere hemmt die erfindungsgemäße Verbindung der Formel I die Thrombozytenaggregation und eignet sich zur Verhinderung von Thrombosen. Weiterhin hemmt die Verbindung der Formel I die Metastasierung von Karzinomzellen. Gegenstand der vorliegend Erfindung ist auch die Verwendung der Verbindung der Formel I und/oder ihrer physiologisch verträglichen Salze für diese Zwecke sowie die Verwendung zur Herstellung von Arzneimitteln zur Hemmung der Thrombozytenaggregation, zur Verhinderung von Thrombosen oder zur Hemmung der Metastasierung von Karzinomzellen.

Die Verbindung der Formel I und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis der Verbindung der Formel I oder eines physiologisch verträglichen Salzes davon neben üblichen, pharmazeutisch einwandfreien Träger- und Zusatzstoffen enthalten. Gegenstand der vorliegenden Erfindung sind auch die Verbindung der Formel I und ihre physiologisch verträglichen Salze zur Verwendung als Arzneimittel sowie pharmazeutische Zubereitungen, die die Verbindung der Formel I und/oder ein oder mehrere physiologisch verträgliche Salze davon als Wirkstoff zusammen mit pharmazeutisch annehmbaren Träger- und Zusatzstoffen und gegebenenfalls noch einen oder mehrere andere pharmakologische Wirkstoffe enthalten. Solche pharmazeutischen Zubereitungen enthalten normalerweise etwa 0.5 bis 90 Gew.% der Verbindung der Formel I oder von physiologisch verträglichen Salzen davon.

Die Arzneimittel können oral, z. B. in Form von Pillen, Tabletten, Filmtabletten, Dragees, Granulaten, Hart- und Weichgelatinekapseln, Lösungen, Sirupen, Emulsionen oder Suspensionen verabreicht werden. Die Verabreichung kann aber auch rektal, z. B. in Form von Suppositorien, oder parenteral, z. B. in Form von Injektionslösungen, Infusionslösungen, Mikrokapseln oder Rods, perkutan, z. B. in Form von Salben oder Tinkturen, oder auf anderem Wege, z. B. in Form von Nasalsprays oder Aerosolmischungen, erfolgen.

Die Herstellung der pharmazeutischen Zubereitungen erfolgt in an sich bekannter Weise, wobei pharmazeutisch inerte anorganische und/oder organische Träger- und Zusatzstoffe verwendet werden und diese zusammen mit den Wirkstoffen in die gewünschte Darreichungsform gebracht werden. Für die Herstellung von Pillen, Tabletten, Dragees und Hartgelatinekapseln kann man z. B. Lactose, Maisstärke oder Derivate davon, Talk, Stearinsäure oder deren Salze, etc. verwenden. Trägerstoffe für Weichgelatinekapseln und Suppositorien sind z. B. Fette, Wachse, halbfeste und flüssige Polyole, natürliche oder gehärtete Öle, etc. Als Trägerstoffe für die Herstellung von Lösungen und Sirupen eignen sich z. B. Wasser, Saccharose, Invertzucker, Glukose, Polyole, etc. Als Trägerstoffe für die Herstellung von Injektionslösungen eignen sich Wasser, Alkohole, Glycerin, Polyole, pflanzliche Öle, etc. Als Trägerstoffe für Mikrokapseln, Implantate oder Rods eignen sich Mischpolymerisate aus Glykolsäure und Milchsäure.

Die pharmazeutischen Zubereitungen können neben den Wirk- und Trägerstoffen noch Zusatzstoffe, wie z. B. Füllstoffe, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Nicotinsäure und ihre Ester, Bencyclan, Naftidrofuryl, Prostacyclinderivate, PGE₁ -Derivate; antianginöse Verbindungen, wie Isosorbiddinitrat, Isosorbidmononitrat, Glycerolnitrat, Molsidomin, Nifedipin und Verapamil; β-Blocker, wie Propranolol, Oxprenolol, Atenolol, Metoprolol und Penbutolol. Darüber hinaus lassen sich die Verbindungen mit ACE-Inhibitoren, wie Captopril, Ramipril, Enalapril, Lisinopril und Trandolapril, Inhibitoren der Thrombozytenfunktion, wie Acetylsalicylsäure, Ticlopidin und Clopidogrel, und Hemmstoffen der Blutgerinnung, wie Heparin und niedermolekularen Heparinen, kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0.1 bis 5 mg/kg, vorzugsweise 0.3 bis 3 mg/kg, insbesondere 0.5 bis 2 mg/kg Körpergewicht zur Erzielung wirksamer Ergebnisse angemessen. Bei intravenöser Applikation beträgt die Tagesdosis im allgemeinen etwa 0.01 bis 0.6 mg/kg, vorzugsweise 0.05 bis 0.3 mg/kg, insbesondere 0.05 bis 0.1 mg/kg Körpergewicht. Die Tagesdosis kann, insbesondere bei der Applikation größerer Mengen, in mehrere, z. B. 2, 3, oder 4 Teilverabreichungen aufgeteilt werden. Gegebenenfalls kann es, je nach individuellem Verhalten, erforderlich werden, von der angegebenen Tagesdosis nach oben oder nach unten abzuweichen. Pharmazeutische Zubereitungen enthalten normalerweise 0.2 bis 500 mg, vorzugsweise 1 bis 200 mg, insbesondere 10 bis 100 mg Wirkstoff der Formel I bzw. von Salzen davon pro Dosis.

Die erfindungsgemäße Verbindung kann zum Beispiel eingesetzt werden bei der Bekämpfung beziehungsweise Vorbeugung von Erkrankungen des kardiovaskulären Systems, Erkrankungen des Koronargefäßsystems oder des cerebrovaskulären Systems, oder von peripheren arteriellen Erkrankungen. Sie finden akut Anwendung bei Thrombosegefahr und chronisch bei der Prävention der Arteriosklerose und Thrombose, z. B. bei der Therapie und Prophylaxe arterieller Gefäßerkrankungen, wie bei akutem Myokardinfarkt, Sekundärprävention des Myokardinfarkts, Reokklusionsprophylaxe nach Lyse und Dilatation (PTCA), instabiler Angina pectoris, transitorischen ischämischen Attacken, Schlaganfall, koronarer Bypass-Operation einschließlich Reokklusionsprophylaxe bei Bypass, Lungenembolie, peripherer arterieller Verschlußkrankheit, Dissezierendem Aneurysma; weiterhin bei der Therapie venöser und mikrozirkulatorischer Gefäßerkrankungen, wie tiefer Venenthrombose, disseminenter intravaskulärer Gerinnung, postoperativem und post-partum Trauma, chirurgischem oder infektiösem Schock, Septicämie oder bei Erkrankungen mit hyperreagiblen Thrombozyten, thrombotischer thrombozytopenischer Purpura, Preeklampsie, prämenstruellem Syndrom, oder bei Dialyse oder extrakorporaler Zirkulation; eine weitere Anwendung ist auch während Krebsoperationen und auch prophylaktisch bei Krebs gegeben. Die Verwendung der erfindungsgemäßen Verbindung der Formel I und/oder ihrer physiologisch verträglichen Salze bei der Behandlung oder Prophylaxe dieser Krankheiten ist Gegenstand der vorliegenden Erfindung, ebenso wie die Verwendung zur Herstellung von Arzneimitteln für die Behandlung oder Prophylaxe der genannten Krankheiten. Insbesondere ist Gegenstand der Erfindung die Verwendung zur Herstellung von Arzneimitteln zur Behandlung oder Vorbeugung von Erkrankungen des Koronargefäßsystems oder des cerebrovaskulären Systems, von peripheren arteriellen Erkrankungen oder von venösen oder mikrozirkulatorischen Gefäßerkrankungen, sowie die Verwendung zur Herstellung von Arzneimitteln, die bei Dialyse oder extrakorporaler Zirkulation eingesetzt werden.

Geprüft wird die Verbindung der Formel I auf ihre hemmende Wirkung bei der Blutplättchenaggregation und bei der Anhaftung von Fibrinogen an Blutplättchen (verwendet werden gelfiltrierte Blutplättchen aus humanem Spenderblut, die mit ADP oder Thrombin aktiviert werden), sowie auf ihre in vivo-Wirkung bei der Hemmung der Thrombozytenaggregation und bei der Thrombosehemmung.

### Beispiele

Alle Produkte wurden über Massenspektren und NMR Spektren identifiziert.

### Beispiel 1

### (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleat

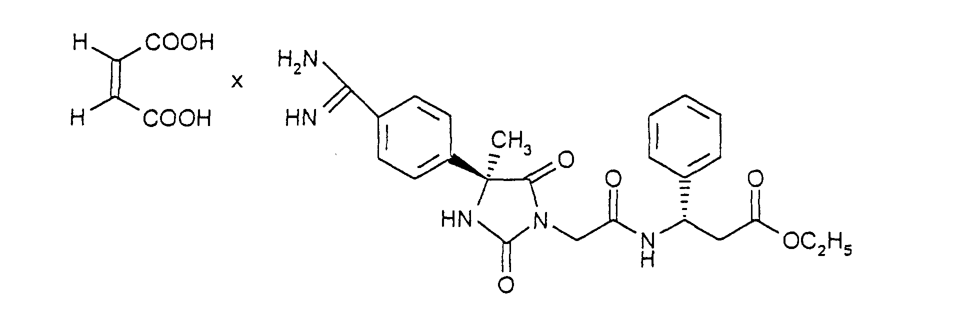

100 g Amberlite® IRA93 (freie Base, Fluka) wurden mit 1000 ml 2 M Maleinsäure-Lösung für 10 min bei Raumtemperatur gerührt. Die Maleinsäure-Lösung wurde abgesaugt und der Ionenaustauscher wurde nochmals zweimal auf die gleiche Weise mit 2 M Maleinsäure-Lösung behandelt. Das Ionenaustauscher-Harz wurde anschließend mit Wasser neutral gewaschen. 1.51 g (3 mmol) (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrochlorid wurden in 15 ml Wasser gelöst. Die Lösung ließ man über eine Ionenaustauscher-Säule laufen, die mit 80 ml Amberlite® IRA93 gefüllt war, der wie vorstehend beschrieben mit Maleinsäure beladen wurde. Die Säule wurde dann mit Wasser eluiert. Es wurden Fraktionen zu je 25 ml gesammelt. Aus den Fraktionen 2 und 3 kristallisierte bei Raumtemperatur reines (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleat aus. Die Kristalle wurden durch Filtration isoliert.
Ausbeute: 771 mg eines weißen, kristallinen Feststoffes (44 %).
Schmp. 228 °C
[α]_{D} = -54.4° (c = 1; Methanol; 22 °C)
FAB-MS: 466 (M + H)⁺
Durch eine Röntgenkristallstrukturanalyse wurde bestätigt, daß es sich um das (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleat handelt (mit einem molaren Verhältnis Ethylester : Maleinsäure von 1:1), das auch durch die folgende ionische Formel wiedergegeben werden kann, die zur obigen Formel äquivalent ist.

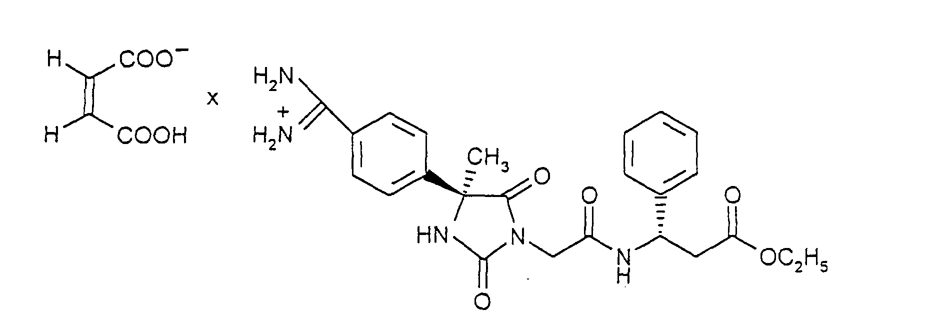

Aus den Mutterlaugen der Fraktionen 2 und 3 und aus den Fraktionen 4 bis 12 ließen sich weitere 740 mg reines (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleat durch Gefriertrocknung der wäßrigen Lösung isolieren. Gesamtausbeute: 1.511 g (87 %).

### Beispiel 2

### (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleat

### 2a. (S)-3-(2-((S)-4-(4-Cyanphenyl)-4-methyl-2,5-dioxo-imidazolidin-1-yl)-acetylamino)-3-phenyl-propionsäure-ethylester

7.7 kg (33.5 mol) (S)-3-Amino-3-phenyl-propionsäure-ethylester-hydrochlorid wurden in einem Kessel vorgelegt und mit einer Lösung von 9.14 kg (33.5 mol) 2-((S)-4-(4-Cyanphenyl)-4-methyl-2,5-dioxo-imidazolidin-1-yl)essigsäure in 50 l Ethylacetat versetzt. Bei einer Innentemperatur von 20 bis 23 °C wurden 7.72 kg (8.54 l; 67 mol) N-Ethylmorpholin zugepumpt. Das Gemisch wurde 15 min gerührt, dann wurden bei 20 bis 23 °C in einer Portion 11 kg (33.5 mol) O-((Cyan(ethoxycarbonyl)methylen)amino-N, N,N',N'-tetramethyluronium-tetrafluoroborat (TOTU) über eine Eintragsschleuse eingetragen. Man rührte 2 h bei einer Temperatur von 20 bis 23 °C nach, dann wurden 50 l Wasser zugesetzt und das Gemisch wurde 30 min bei einer Temperatur von 20 bis 23 °C gerührt. Es wurden 50 l Methyl-tert.-butylether zugesetzt, und das Gemisch wurde anschließend 2 h bei einer Temperatur von 8 bis 12 °C gerührt. Die weiße Suspension wurde in eine Zentrifuge überführt und zentrifugiert. Der Filterkuchen wurde mit 28 l Wasser, anschließend mit 28 l Ethanol und abschließend nochmals mit 56 l Wasser gewaschen. Das Produkt wurde im Vakuumtrockenschrank bei 40 °C im Stickstoffstrom getrocknet.
Ausbeute: 9.84 kg (85 %).
FAB-MS: 449 (M + H)⁺

### 2b. (S)-3-(2-((S)-4-(4-(Amino-hydroximino-methyl)-phenyl)-4-methyl-2,5-dioxo-imidazolidin-1-yl)-acetylamino)-3-phenyl-propionsäure-ethylester

120 l Ethanol wurden in einem Kessel vorgelegt. Bei Raumtemperatur wurden 12 kg (26.786 mol) (S)-3-(2-((S)-4-(4-Cyanphenyl)-4-methyl-2,5-dioxo-imidazolidin-1-yl)-acetylamino)-3-phenyl-propionsäure-ethylester unter Rühren in den Kessel gegeben. Man setzte 3.74 kg (53.813 mol) Hydroxylamin-hydrochlorid und 5.44 kg (7.45 l; 53.86 mol) Triethylamin zu. Das Reaktionsgemisch wurde zum Rückfluß erwärmt und 2 h bei 80 °C gerührt. Bei einer Badtemperatur von 50 °C wurden 90 l Ethanol im Vakuum abdestilliert. Der Rückstand wurde in 220 l Ethylacetat aufgenommen und dreimal mit jeweils 50 I Wasser extrahiert. Die Ethylacetat-Phase wurde im Vakuum bei einer Badtemperatur von 50 °C bis auf 30 l eingedampft. Es wurde viermal mit je 10 l Toluol versetzt und bis zur Trockene eingedampft.
Ausbeute: 11.1 kg (85 %).
FAB-MS: 482 (M + H)⁺

### 2c. (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Essigsäuresalz

11 kg (22.869 mol) (S)-3-(2-((S)-4-(4-(Amino-hydroximino-methyl)-phenyl)-4-methyl-2,5-dioxo-imidazolidin-1-yl)-acetylamino)-3-phenyl-propionsäure-ethylester wurden in 70 l Eisessig gelöst und in einen 125 I-Autoklaven gegeben. Zu der Lösung gab man eine Suspension aus 1.0 kg Palladium-Kohle (10 %ig; 50 % Wasser) und 5 l Eisessig. Es wurde bei 50 °C und 10 bar Wasserstoffdruck für 15 h hydriert. Der Katalysator wurde über eine Drucknutsche unter Stickstoff abgesaugt und mit 5 l Eisessig gewaschen. Das Filtrat wurde bei einer Badtemperatur von 60 °C im Vakuum am Rotationsverdampfer vollständig eingedampft. Der Rückstand wurde in 30 l Aceton gelöst und die Lösung wurde innerhalb von 30 min bei 20 °C zu 200 l Methyl-tert.-butylether zugegeben. Es wurde 1 h bei Raumtemperatur nachgerührt und das ausgefallene Produkt geschleudert. Der Filterkuchen wurde mit 10 l Methyltert.-butylether nachgewaschen, ausgetragen und im Vakuum getrocknet. Man erhielt 10.1 kg (85 %) Produkt. FAB-MS: 466 (M + H)^{+.}

Zur Reinigung wurden in einem Kessel 90 l Aceton vorgelegt und 10.1 kg des obigen Produktes zugegeben. Es wurde auf 50°C erwärmt und 1 h gerührt. Bei einer Manteltemperatur von 20 °C wurde über Nacht weitergerührt und der Feststoff über eine Schleuder isoliert. Es wurde mit 10 l Aceton gewaschen. Das acetonfeuchte Produkt wurde mit 50 l Aceton verrührt und auf Rückflußtemperatur (57 °C) erwärmt und 1 h gerührt. Man ließ unter Rühren über 24 h abkühlen, rührte 1 h bei 15 °C und isolierte das Produkt über eine Zentrifuge. Der Filterkuchen wurde mit 10 l Methyl-tert.-butylether gewaschen, ausgetragen und im Vakuum bei 0.1 bar und 40 °C getrocknet. Das Produkt wurde gemahlen.
Ausbeute: 7.2 kg (60 %).
FAB-MS: 466 (M + H)⁺

### 2d. (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleat

75.68 g (144 mmol) (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Essigsäure-Salz wurden in 340 ml Wasser gelöst. Die Lösung wurde filtriert und unter Rühren mit einer Lösung von 16.87 g (144 mmol) Maleinsäure (99 %ig) in 48 ml Wasser versetzt. Man rührte für 1 h bei Raumtemperatur und ließ anschließend über Nacht im Eisbad stehen. Das Produkt fiel als kristalliner Feststoff aus der Lösung aus. Man saugte ab und trocknete im Hochvakuum über Phosphorpentoxid.
Ausbeute: 80.7 g eines weißen, kristallinen Feststoffes (96 %).
Schmp. 228 °C
[α]_{D} = -54.4 ° (c = 1; Methanol; 22 °C)
FAB-MS: 466 (M + H)⁺

### Herstellung der in Schritt 2 a eingesetzten Ausgangsverbindungen

### I. (S)-3-Amino-3-phenyl-propionsäure-ethylester-hydrochlorid

### Ia. (R)-2-Amino-2-phenylethanol

20 g (920 mmol) Lithiumborhydrid wurden in 420 ml absolutem Tetrahydrofuran gelöst. Man tropfte unter Rühren 233.5 ml (1.84 mol) Trimethylchlorsilan zu und setzte anschließend portionsweise innerhalb von 4 Stunden 69.5 g (0.46 mol) (R)-Phenylglycin zu. Das Reaktionsgemisch wurde über Nacht bei Raumtemperatur gerührt. Dann setzte man 690 ml Methanol zu, rührte für 2 Stunden bei Raumtemperatur und engte im Vakuum ein. Der Rückstand wurde unter Rühren in 690 ml 20 %iger wäßriger Kaliumhydroxid-Lösung gelöst. Die wäßrige Phase wurde dreimal mit Essigsäureethylester extrahiert. Die vereinigten organischen Phasen wurden mit Wasser gewaschen, über Magnesiumsulfat getrocknet und im Vakuum eingeengt.
Ausbeute: 41.2 g (65.3 %).
FAB-MS: 138 (M + H)⁺

### Ib. (R)-2-Benzyloxycarbonylamino-2-phenylethanol

40.5 g (295 mol) (R)-2-Amino-2-phenylethanol wurden in 385 ml absolutem Dimethylformamid gelöst. Man setzte unter Rühren bei 0 °C 73.5 g N-(Benzyloxycarbonyl-oxy)-succinimid (295 mmol) zu und rührte für 1 Stunde bei 0 °C. Das Eisbad wurde entfernt und der Ansatz für 48 h bei Raumtemperatur stehen gelassen. Die Reaktionslösung wurde im Vakuum eingeengt und der Rückstand anschließend in 500 ml Essigsäureethylester aufgenommen. Die organische Phase wurde zweimal mit 10 %iger wäßriger Citronensäure-Lösung sowie einmal mit Wasser gewaschen. Man trocknete über wasserfreiem Natriumsulfat und engte ein. Das erhaltene kristalline Rohprodukt (82.3 g) wurde erneut in Essigsäureethylester gelöst. Die organische Phase wurde zweimal mit 10 %iger wäßriger Citronensäure-Lösung sowie einmal mit Wasser gewaschen. Anschließend kristallisierte man aus Essigsäureethylester/Petrolether um.
Ausbeute: 74.6 g (93.3 %).
FAB-MS: 272 (M + H)⁺

### Ic. ((R)-2-Benzyloxycarbonylamino-2-phenyl-ethyl)-4-methylphenylsulfonat

53.9 g (R)-2-Benzyloxycarbonylamino-2-phenylethanol (198.7 mmol) wurden in einer Mischung aus 500 ml Methylenchlorid sowie 80.3 ml (993.5 mmol) Pyridin gelöst. Man setzte unter Rühren bei 0 °C 45.5 g (238.4 mmol) Tosylchlorid in 240 ml Methylenchlorid zu und ließ 7 Stunden bei Raumtemperatur rühren. Es wurden weitere 11.36 g Tosylchlorid (59.61 mmol) zugesetzt. Man ließ 5 Stunden bei 0 °C rühren. Der Ansatz wurde dann über Nacht bei Raumtemperatur stehen gelassen und im Vakuum eingeengt. Der Rückstand wurde in Essigsäureethylester aufgenommen. Die organische Phase wurde dreimal mit 10 %iger wäßriger Citronensäure-Lösung und zweimal mit Wasser gewaschen, über Magnesiumsulfat getrocknet und im Vakuum eingeengt. Der Rückstand wurde mit Diethylether verrieben, abgesaugt, mit Diethylether gewaschen und über Phosphorpentoxid getrocknet. Ausbeute: 60.9 g (72 %). Die Mutterlauge wurde eingeengt, in n-Heptan/Essigsäureethylester (6 : 4) aufgenommen und über Kieselgel chromatographiert. Ausbeute: 3.5 g (4.2 %).
Gesamtausbeute: 64.4 g (76.2 %).
FAB-MS: 426 (M + H)⁺

### Id. (S)-3-Benzyloxycarbonylamino-3-phenyl-propionitril

60.5 g ((R)-2-Benzyloxycarbonylamino-2-phenyl-ethyl)-4-methylphenylsulfonat (142.2 mmol) wurden in 675 ml Dimethylformamid gelöst. Man setzte 13.9 g Kaliumcyanid (213.3 mmol), 5.64 g 18-Krone-6 (21.33 mmol) und 520 mg Kaliumiodid (3.13 mmol) zu und rührte 20 Stunden bei 50 °C. Die Reaktionslösung wurde in 500 ml Eiswasser gegossen und anschließend 5 Stunden bei 0 °C gerührt. Man saugte ab und löste den Niederschlag in Essigsäureethylester. Die organische Phase wurde dreimal mit Wasser gewaschen, über Magnesiumsulfat getrocknet und im Vakuum eingeengt. Der Rückstand wurde mit Diethylether verrieben, abgesaugt, mit Diethylether gewaschen und über Phosphorpentoxid getrocknet.
Ausbeute: 25.3 g (63.5 %).
FAB-MS: 281 (M + H)⁺

### le. (S)-3-Benzyloxycarbonylamino-3-phenyl-propionsäureethylester

15 g (S)-3-Benzyloxycarbonylamino-3-phenyl-propionitril (53.51 mmol) wurden in einer Mischung aus 110 ml absolutem Ethanol und 30 ml Dioxan suspendiert. Unter Rühren und Kühlung leitete man bei 10 - 15 °C HCl-Gas ein. Nach kurzer Zeit bildete sich eine klare Lösung. Man leitete weiter HCl-Gas unter Kühlung ein, bis im Dünnschichtchromatogramm kein Ausgangsmaterial mehr nachgewiesen werden konnte. Es wurde dann für 15 Minuten Stickstoff durch die Reaktionslösung geleitet und anschließend im Vakuum eingeengt. Der Rückstand wurde bis zur bleibenden Trübung mit Wasser versetzt. Man rührte 30 Minuten bei Raumtemperatur und extrahierte anschließend die wäßrige Phase dreimal mit Essigsäureethylester. Die vereinigten organischen Phasen wurden mit Wasser gewaschen, über Magnesiumsulfat getrocknet und im Vakuum eingeengt. Der Rückstand wurde in Essigsäureethylester/Petrolether (1 : 1) aufgenommen und über Kieselgel chromatographiert.
Ausbeute: 10.55 g (60 %).
FAB-MS: 328 (M + H)⁺

### If. (S)-3-Amino-3-phenyl-propionsäure-ethylester-hydrochlorid

10.29 g (S)-Benzyloxycarbonylamino-3-phenyl-propionsäure-ethylester (31.44 mmol) wurden in 125 ml Ethanol gelöst und an der Autobürette unter Zugabe von 2 N ethanolischer HCl bei einem pH von 4 über Pd/Aktivkohle katalytisch hydriert. Der Katalysator wurde über Kieselgur abgesaugt und das Filtrat eingeengt. Der Rückstand wurde mit Diethylether verrieben, abgesaugt, mit Diethylether gewaschen und über Phosphorpentoxid getrocknet.
Ausbeute: 5.05 g (70 %).
FAB-MS: 194 (M + H)⁺

### II. 2-((S)-4-(4-Cyanophenyl)-4-methyl-2,5-dioxoimidazolidin-1-yl)essigsäure

### IIa. (R, S)-4-(4-Bromophenyl)-4-methyl-2,5-dioxoimidazolidin

49.8 g (0.25 mol) 4-Bromacetophenon, 21.2 g (0.325 mol) Kaliumcyanid und 211.4 g (2.2 mol) Ammoniumcarbonat wurden in 1.0 l einer wäßrigen Ethanol-Lösung (0.5 l destilliertes Wasser und 0.5 l Ethanol) suspendiert. Die Suspension wurde bei 60 °C gerührt, bis sich dünnschichtchromatographisch kein Ausgangsmaterial mehr nachweisen ließ (8 Stunden). Man ließ auf Raumtemperatur abkühlen. Der pH der Lösung wurde mit halbkonzentrierter Salzsäure auf pH = 6.3 eingestellt. Das Produkt fiel als weißer Niederschlag aus. Der Ansatz wurde über Nacht bei 4 °C stehen gelassen. Der weiße Niederschlag wurde abgesaugt, mit Wasser gewaschen und im Vakuum über Phosphorpentoxid getrocknet.
Ausbeute: 65.3 g eines weißen Feststoffes (97 %).
FAB-MS: 269 (M + H)⁺

### IIb. (R, S)-2-Amino-2-(4-bromophenyl)propionsäure

5.3 g (20 mmol) (R,S)-4-(4-Bromophenyl)-4-methyl-2,5-dioxoimidazolidin wurden in 50 ml 3 N Natronlauge suspendiert. Die Suspension wurde im Autoklaven 1 Stunde bei 145 °C bei einem Stickstoffüberdruck von 10 bar erhitzt. Die abgekühlte Reaktionslösung wurde mit 150 ml Wasser verdünnt und unter starkem Rühren mit Essigsäure unter Eiskühlung auf einen pH von 4 gebracht. Es wurde bei 0 °C für 2 Stunden gerührt. Der Niederschlag wurde abgesaugt, mit Wasser gewaschen und im Vakuum über Phosphorpentoxid getrocknet.
Ausbeute: 3.65 g eines weißen Feststoffes (75 %).
FAB-MS: 244 (M + H)⁺

### IIc. (R, S)-2-Amino-2-(4-bromophenyl)propionsäureethylester

27.3 g (112.3 mmol) (R,S)-2-Amino-2-(4-bromophenyl)propionsäure wurden in 150 ml 9.8 N ethanolischer Chlorwasserstoff-Lösung suspendiert. Man erhitzte 18 Stunden am Rückfluß, setzte nochmals 50 ml 9.8 N ethanolische Chlorwasserstoff-Lösung zu und erhitzte weitere 5 Stunden am Rückfluß. Die Lösung wurde eingeengt und der Rückstand zwischen Essigsäureethylester und gesättigter Natriumbicarbonat-Lösung verteilt. Die organische Phase wurde mit Wasser gewaschen und über Natriumsulfat getrocknet. Das Rohprodukt (23.22 g) wurde im Hochvakuum zur Reinigung destilliert (Siedepunkt = 129 - 130 °C bei 2 Torr).
Ausbeute: 20.7 g (68 %).
FAB-MS: 272 (M + H)⁺

### IId. (S)-2-Amino-2-(4-bromophenyl)propionsäureethylester

44.3 g (163 mmol) (R,S)-2-Amino-2-(4-bromophenyl)propionsäureethylester und 24.8 g D-(-)-Mandelsäure (163 mmol) wurden in 138 ml Isopropanol bei Raumtemperatur gelöst. Man setzte 414 ml Diisopropylether zu und kühlte über Nacht bei 0 °C. Der ausgefallene Niederschlag wurde abgesaugt. Das erhaltene Salz wurde noch zwei weitere Male in der gleichen Weise umkristallisiert. Es wurden 20 g enantiomerenreines Salz erhalten ([α]_{D} = - 14 ° (c = 1; 2.15 N ethanolische Chlorwasserstoff-Lösung; 22 °C). Das Salz wurde zwischen Essigsäureethylester und wäßriger Natriumbicarbonat-Lösung verteilt. Die organische Phase wurde mit Wasser gewaschen, über Magnesiumsulfat getrocknet und im Vakuum eingeengt. Die Enantiomerenreinheit wurde nach Derivatisierung mit R-(-)-α-Methoxy-α-(trifluormethyl)-phenylessigsäurechlorid (Mosher-Reagenz) durch HPLC zu größer 99 % ee bestimmt.
Ausbeute: 12.5 g (28 %).
[α]_{D} = + 52.7 ° (c = 1; 2.15 N ethanolische Chlorwasserstoff-Lösung; 22 °C)
FAB-MS: 272 (M + H)⁺

### IIe. N-((S)-1-(4-Bromophenyl)-1-(ethoxycarbonyl)ethyl)-N'-(ethoxycarbonyl-methyl)harnstoff

12.4 g (45.6 mmol) (S)-2-Amino-2-(4-bromophenyl)propionsäureethylester wurden in 70 ml Methylenchlorid gelöst. Man tropfte bei 0 °C innerhalb von 15 Minuten eine Lösung von 5.11 ml (45.6 mmol) Isocyanatoessigsäure-ethylester in 35 ml Methylenchlorid zu. Es wurde 2 Stunden bei 0 °C gerührt und dann eingeengt.
Ausbeute: 18.1 g (99 %).
[α]_{D} = + 10.7 ° (c = 1; 2.15 N ethanolische Chlorwasserstoff-Lösung; 22 °C)
FAB-MS: 401 (M + H)⁺

### IIf. 2-((S)-4-(4-Bromophenyl)-4-methyl-2,5-dioxoimidazolidin-1-yl)essigsäure

18 g (44.9 mmol) N-((S)-1-(4-Bromophenyl)-1-(ethoxycarbonyl)ethyl)-N'-(ethoxycarbonylmethyl)harnstoff wurden mit 180 ml 6 N Chlorwasserstoff-Lösung versetzt. Das Reaktionsgemisch wurde 10 Stunden unter Rückfluß zum Sieden erhitzt. Man ließ auf 0 °C abkühlen und saugte das ausgefallene Reaktionsprodukt ab. Es wurde mit Wasser nachgewaschen und im Vakuum über Phosphorpentoxid getrocknet.
Ausbeute: 11.4 g (78 %).
[α]_{D} = + 32.8 ° (c = 1; 2.15 N ethanolische Chlorwasserstoff-Lösung; 22 °C)
FAB-MS: 327 (M + H)⁺

### IIg. 2-((S)-4-(4-Cyanophenyl)-4-methyl-2,5-dioxoimidazolidin-1-yl)essigsäure

11.75 g (35.9 mmol) 2-((S)-4-(4-Bromophenyl)-4-methyl-2,5-dioxoimidazolidin-1-yl)essigsäure wurden in 90 ml Dimethylformamid gelöst. Man setzte 14.15 g (158 mmol) Kupfer-I-cyanid zu und erhitzte 20 Stunden unter Rühren am Rückfluß. Das Reaktionsgemisch wurde abgekühlt und dann in 300 ml Wasser gegossen. Die wäßrige Phase wurde mit konzentrierter Salzsäure sauer gestellt (pH = 1 - 1.5), 30 Minuten gerührt und über eine Seitzschicht abgesaugt. Die wäßrige Phase wurde dreimal mit Essigsäureethylester extrahiert. Die vereinigten organischen Phasen wurden mit Wasser gewaschen, über Magnesiumsulfat getrocknet und im Vakuum eingeengt.
Ausbeute: 9.3 g (95 %).
[α]_{D} = + 33.4 ° (c = 1; 2.15 N ethanolische Chlorwasserstoff-Lösung; 22 °C)
FAB-MS: 274 (M + H)⁺

### Beispiel 3

### Bestimmung der Wasserdampfsorption

Die Bestimmung der Wasseraufnahme einer Substanz erfolgte mit Hilfe einer automatischen zweischenkeligen Mikrowaage, die im Vakuum bzw. in einer Atmosphäre betrieben wird, die eine definierte Zusammensetzung bzw. einen definierten Druck hat. Auf einer Seite der Waage befindet sich die trockene Probe, auf der anderen Seite ein Tariergewicht. Im Wägeraum wurde eine Wasserdampfatmosphäre erzeugt, deren Druck schrittweise erhöht wurde. Die Gewichtszunahme der Probe wurde in Abhängigkeit vom Wasserdampfdruck registriert. Die Messung erfolgte bei einer konstanten Temperatur von 25 °C.

Der Wägeraum wurde zunächst bis zur Gewichtskonstanz evakuiert und das Ausgangsgewicht der Probe bestimmt. Dann wurde in den evakuierten Wägeraum durch ein Einlaßventil eine bestimmte Menge Wasserdampf eingeleitet. In dem unten wiedergegebenen Versuch entsprach der in diesem ersten Schritt eingestellte Wasserdampfdruck bei der Meßtemperatur von 25 °C einer relativen Luftfeuchtigkeit von 4.7 %. Wenn nach dem Einleiten des Wasserdampfs wieder Gewichtskonstanz erreicht war, d. h. wenn die Probe im Gleichgewicht mit der Wasserdampfatmosphäre war, wurde die Gewichtszunahme der Probe registriert. Dann wurde in mehreren Schritten durch Einleiten weiteren Wasserdampfs der Wasserdampfdruck erhöht (jeder Wasserdampfdruck entspricht dabei einer bestimmten relativen Luftfeuchtigkeit bei der Meßtemperatur von 25 °C). Bei jedem Schritt wurde nach Erreichen der Gewichtskonstanz die Gewichtszunahme der Probe registriert. Bei der Untersuchung von (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleat wurden folgende Gewichtszunahmen, bezogen auf das Ausgangsgewicht der Probe, beobachtet.

| | | | | | | | |
|---|---|---|---|---|---|---|---|
| Relative Feuchtigkeit | 4.7 % | 18.5 % | 32.8 % | 49.6 % | 64.7 % | 75.5 % | 82.1 % |
| Gewichtszunahme der Probe | 0.06 % | 0.11 % | 0.16 % | 0.23 % | 0.33 % | 0.43 % | 0.54 % |

Die Ergebnisse zeigen, daß selbst bei einer relativen Luftfeuchtigkeit von ca. 80 % die Gewichtszunahme des (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleats nur sehr gering ist (ca. 0.5 %), die Substanz also nicht hygroskopisch ist.

### Beispiel 4

### Pharmakologische Untersuchung im Modell Zyklische Schwankungen der Koronardurchblutung am Hund (Modell der instabilen Angina pectoris)

Hunde beiderlei Geschlechts (20 - 35 kg) wurden durch Infusion von Ketamin und Natriumpentobarbital narkotisiert, künstlich beatmet und für die Messung der hämodynamischen Parameter (peripherer Blutdruck, linksventrikulärer Druck, Kontraktilität des Herzmuskels, Herzfrequenz) sowie für die Aufzeichnung des EKG vorbereitet. Blutgase und Hämatokrit wurden durch Infusion von Elektrolyten konstant gehalten. Nach Eröffnung der linken Brusthöhle wurde das Herz freigelegt. Der Ramus circumflexus sin. der Koronararterie wurde zur Bestimmung der mittleren Koronardurchblutung mit einem elektromagnetischen Durchflußmeßkopf versehen. Distal zu diesem Meßkopf wurde der Ramus circumflexus zur Schädigung des Gefäßendothels kurzfristig mit einer Klemme zusammengedrückt. Im geschädigten Bereich wurde mit einem kleinen Kunststoffzylinder (Innendurchmesser je nach Gefäßgröße 1.0 - 1.7 mm, Länge 4 mm) eine konzentrische Konstriktion hervorgerufen. Dadurch kommt es zur Unterdrückung der reaktiven Hyperämie im Gefolge einer 10 Sekunden langen Okklusion des Ramus circumflexus und zur Entstehung zyklischer Durchblutungsschwankungen (ca. 10 pro Stunde) aufgrund der wiederholten Bildung thrombozytenreicher Thromben im Gebiet der Stenose. 60 Minuten nach dem ersten Auftreten regelmäßiger Schwankungen des Blutflusses wurden die Prüfsubstanzen als intravenöse oder intraduodenale Bolus-Verabreichung appliziert. Anschließend wurden die zyklischen Durchblutungsschwankungen mindestens 2 - 5 Stunden lang beobachtet. Die prozentuale Hemmung der koronarthrombotischen zyklischen Durchblutungsschwankungen wurde an 5 - 8 Tieren durch einen Vergleich zwischen der durchschnittlichen Zahl von Durchblutungsschwankungen pro Stunde vor und nach Verabreichung der Prüfsubstanzen ermittelt. Bei den Kontrolltieren blieb die Anzahl zyklischer Durchblutungsschwankungen pro Stunde mehrere Stunden lang konstant. (Literatur: Folts, J. D. et al. (1976), Circulation 54: 365 - 370; Just, M. et al. (1989), J. Cardiovasc. Pharmacol. 14 (Suppl. 11): S 129 - 136)

Bei der Untersuchung von (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-hydrogenmaleat wurden die folgenden Ergebnisse erhalten. Angegeben ist die prozentuale Verringerung der Anzahl der Stenose-induzierten zyklischen Schwankungen des koronaren Blutflusses pro Stunde in der ersten, zweiten und dritten Stunde nach der Verabreichung der Substanz im Vergleich zum Zeitraum vor der Verabreichung. Die Dosis ist in mg pro kg Körpergewicht angegeben.

| Dosis (mg/kg) | Verabreichungsart | Verringerung der zyklischen Schwankungen (%) | | |
|---|---|---|---|---|
| | | erste Stunde | zweite Stunde | dritte Stunde |
| 0.1 | intravenös | 52 | 81 | 89 |
| 0.2 | intravenös | 75 | 96 | 99 |
| 0.1 | intraduodenal | 39 | 62 | 74 |
| 0.3 | intraduodenal | 23 | 66 | 78 |

Die in diesem in vivo-Versuch erhaltenen Ergebnisse belegen die starke antithrombotische Aktivität der Prüfsubstanz, insbesondere die koronarantithrombotische Wirkung. Alle während des Versuchs aufgezeichneten hämodynamischen Parameter zeigten keine signifikanten Veränderungen.

### Beispiel 5

### Pharmakologische Untersuchung im Modell Ex-vivo-Hemmung der Thrombozytenaggregation am Hund

Hunden beiderlei Geschlechts (Labrador/Harrier-Mischlinge, Körpergewicht 21 - 28 kg) wurde über Nacht die Nahrung entzogen. Die Prüfsubstanzen wurden oral in Gelatinekapseln oder intravenös verabreicht. Vor Verabreichung der Prüfsubstanzen sowie mehrmals im Zeitraum von 24 Stunden danach wurden 20 ml Blut aus der Vena cephalica entnommen und mit saurer Citratdextrose antikoaguliert (9 + 1 Volumina). Durch Zentrifugation wurde plättchenreiches Plasma (PRP) hergestellt. Die Thrombozytenaggregation in PRP wurde nach Zusatz von ADP (3 - 30 µmol/l) oder Kollagen (0.3 - 10 µg/ml) + Epinephrin (10 µM) bei 37 °C mit einem Aggregometer (BioData) bestimmt. Verglichen wurde die in Prozent ausgedrückte maximale Thrombozytenaggregation vor und nach Gabe der Prüfsubstanzen. Angegeben ist die prozentuale Hemmung der Thrombozytenaggregation bei der niedrigsten Konzentration des Agonisten, durch die noch eine irreversible maximale Aggregation ausgelöst wurde. Ferner wurde bei der Entnahme der Blutproben an der enthaarten Innenseite der Vorderpfote mittels eines Simplate® 1-Geräts die kutane Blutungszeit gemessen. Berechnet wurde die prozentuale Verlängerung der Blutungszeit nach Verabreichung der Prüfsubstanzen im Vergleich zum Ausgangswert.

Bei der Untersuchung von (S)-3-(2-((S)-4-(4-(Amino-imino-methyl)-phenyl)-4-methyl-2,5-dioxo-imidazolidin-1-yl)-acetylamino)-3-phenyl-propionsäure-ethylesterhydrogenmaleat wurden die folgenden Ergebnisse erhalten. Angegeben ist die prozentuale Hemmung der Thrombozytenaggregation 1 h, 4 h, 8 h und 24 h nach der Verabreichung der Substanz. Die Dosis ist in mg pro kg Körpergewicht angegeben. n ist die Zahl der untersuchten Hunde.
a) Hemmung der ADP-induzierten ex vivo-Thrombozytenaggregation

| Dosis (mg/kg) | Verabreichungsart (n) | Hemmung der Aggregation (%) | | | |
|---|---|---|---|---|---|
| | | nach 1 h | nach 4 h | nach 8 h | nach 24 h |
| 0.5 | intravenös (6) | 93 | 88 | 59 | 16 |
| 0.5 | oral (6) | 46 | 83 | 33 | 18 |
| 1 | oral (6) | 76 | 97 | 86 | 70 |
| 2 | oral (8) | 64 | 86 | 78 | 49 |

b) Hemmung der Kollagen-induzierten ex vivo-Thrombozytenaggregation

| Dosis (mg/kg) | Verabreichungsart (n) | Hemmung der Aggregation (%) | | | |
|---|---|---|---|---|---|
| | | nach 1 h | nach 4 h | nach 8 h | nach 24 h |
| 0.5 | intravenös (6) | 27 | 26 | 18 | 0 |
| 0.5 | oral (6) | 8 | 19 | 5 | 0 |
| 1 | oral (6) | 36 | 67 | 50 | 11 |
| 2 | oral (8) | 28 | 65 | 60 | 20 |

Die maximalen Veränderungen der Blutungszeit waren eine Verlängerung um 68 % (p < 0.05) 4 Stunden nach der intravenösen Verabreichung von 0.5 mg/kg und eine Verlängerung um 122 % (p < 0.05) 2 Stunden nach der oralen Verabreichung von 2 mg/kg. Bei den niedrigeren oral verabreichten Dosierungen war die Blutungszeit nicht signifikant verändert. Die Ergebnisse dieses Versuchs belegen die ausgeprägte Wirksamkeit der Prüfsubstanz als in vivo-Inhibitor der Thrombozytenaggregation.

## Patentansprüche

1. 3-(2-(4-(4-(Amino-imino-methyl)-phenyl)-4-methyl-2,5-dioxo-imidazolidin-1-yl)-acetylamino)-3-phenyl-propionsäure-ethylester-Hydrogenmaleat der Formel I, 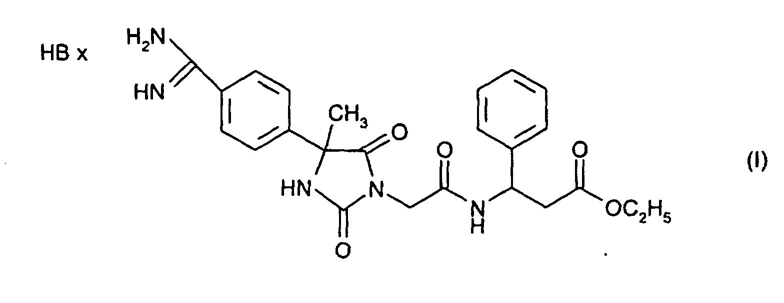 worin HB Maleinsäure bedeutet und am chiralen Zentrum im Imidazolidinring die (S)-Konfiguration und am chiralen Zentrum in der Propionsäureeinheit die (S)-Konfiguration vorliegt, und seine physiologisch verträglichen Salze.

2. (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat.

3. Verfahren zur Herstellung von (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat nach Anspruch 1 oder 2, **dadurch gekennzeichnet, daß** mit Verbindungen der allgemeinen Formel II, 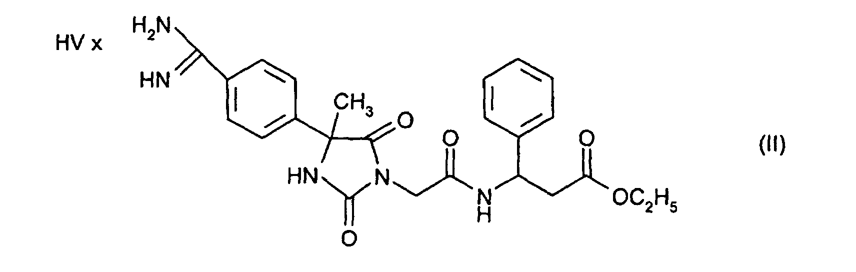 in der HV für eine beliebige anorganische oder organische, von Maleinsäure verschiedene Säure steht und am chiralen Zentrum im lmidazolidinring die (S)-Konfiguration und am chiralen Zentrum in der Propionsäureeinheit die (S)-Konfiguration vorliegt, ein Anionenaustausch mit Maleinsäure und/oder Maleaten durchgeführt wird.

4. Verfahren gemäß Anspruch 3, **dadurch gekennzeichnet, daß** für den Anionenaustausch Verbindungen der allgemeinen Formel II über einen mit Maleinsäure beladenen Ionenaustauscher chromatographiert werden.

5. Verfahren gemäß Anspruch 3, **dadurch gekennzeichnet, daß** für den Anionenaustausch Verbindungen der allgemeinen Formel II in einem Lösungsmittel mit Maleinsäure oder Maleaten vereinigt werden.

6. Verfahren gemäß einem oder mehreren der Ansprüche 3 bis 5, **dadurch gekennzeichnet, daß** der Anionenaustausch mit dem Essigsäuresalz der Formel IIb, 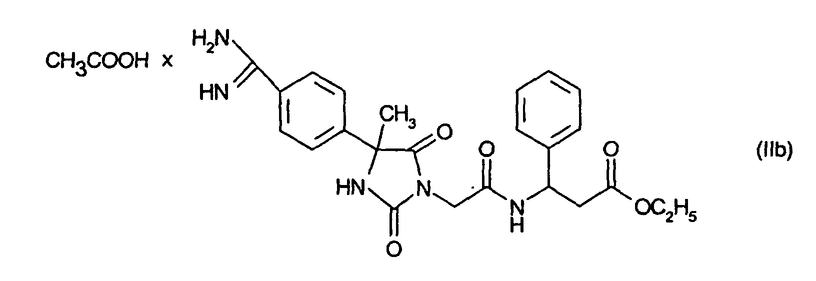 worin am chiralen Zentrum im Imidazolidinring die (S)-Konfiguration und am chiralen Zentrum in der Propionsäureeinheit die (S)-Konfiguration vorliegt, durchgeführt wird.

7. Verfahren gemäß einem oder mehreren der Ansprüche 3 bis 6, **dadurch gekennzeichnet, daß** zur Herstellung der Verbindungen der allgemeinen Formel II die Verbindung der Formel III 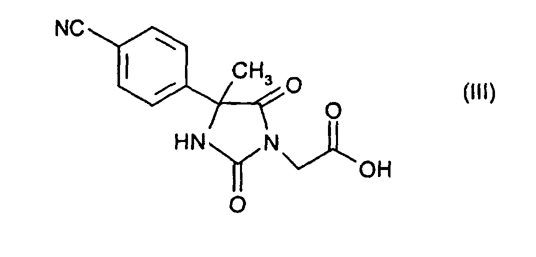 und die Verbindung der Formel IV, 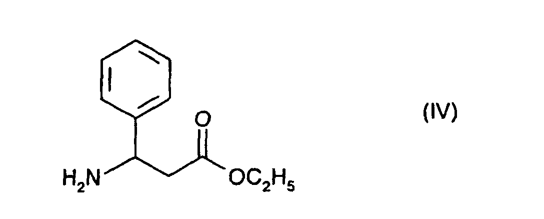 zur Verbindung der Formel V 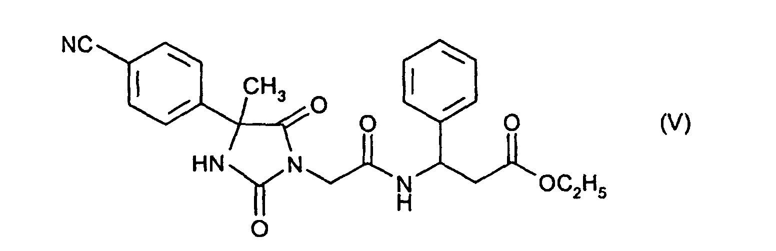 gekuppelt werden, diese mit Hydroxylamin in die Verbindung der Formel VI 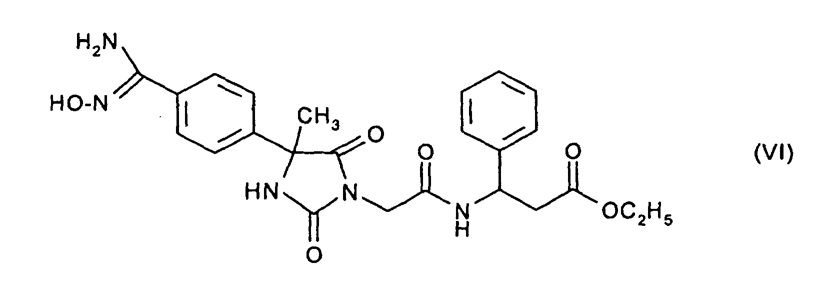 überführt wird und diese durch Hydrierung und Zusatz der Säure der allgemeinen Formel HV in die Verbindungen der allgemeinen Formel II, bevorzugt durch Hydrierung in Gegenwart von Essigsäure in die Verbindung der Formel IIb, überführt werden, wobei in den Verbindungen der Formeln III, IV, V und VI am chiralen Zentrum im Imidazolidinring die (S)-Konfiguration und am chiralen Zentrum in der Propionsäureeinheit die (S)-Konfiguration vorliegt.

8. (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat nach Anspruch 1 und/oder 2 und/oder seine physiologisch verträglichen Salze zur Verwendung als Arzneimittel.

9. (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat nach Anspruch 1 und/oder 2 und/oder seine physiologisch verträglichen Salze zur Verwendung als Hemmstoffe der Thrombozytenaggregation, zur Verwendung bei der Verhinderung von Thrombosen oder zur Verwendung als Hemmstoffe der Metastasierung von Karzinomzellen.

10. Verwendung von (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat nach Anspruch 1 und/oder 2 und/oder seinen physiologisch verträglichen Salzen zur Herstellung von Arzneimitteln zur Hemmung der Thrombozytenaggregation, zur Verhinderung von Thrombosen oder zur Hemmung der Metastasierung von Karzinomzellen.

11. (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat nach Anspruch 1 und/oder 2 und/oder seine physiologisch verträglichen Salze zur Behandlung oder Vorbeugung von Erkrankungen des Koronargefäßsystems oder des cerebrovaskulären Systems, von peripheren arteriellen Erkrankungen oder von venösen oder mikrozirkulatorischen Gefäßerkrankungen, oder zur Verwendung bei Dialyse oder extrakorporaler Zirkulation.

12. Verwendung von (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat nach Anspruch 1 und/oder 2 und/oder seinen physiologisch verträglichen Salzen zur Herstellung von Arzneimitteln zur Behandlung oder Vorbeugung von Erkrankungen des Koronargefäßsystems oder des cerebrovaskulären Systems, von peripheren arteriellen Erkrankungen oder von venösen oder mikrozirkulatorischen Gefäßerkrankungen, oder Arzneimitteln, die bei Dialyse oder extrakorporaler Zirkulation eingesetzt werden.

13. Pharmazeutische Zubereitung, **dadurch gekennzeichnet, daß** sie (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat nach Anspruch 1 und/oder 2 und/oder ein oder mehrere physiologisch verträgliche Salze davon als Wirkstoff zusammen mit pharmazeutisch annehmbaren Träger- und Zusatzstoffen und gegebenenfalls noch einen oder mehrere andere pharmakologische Wirkstoffe enthält.

14. Verfahren zur Herstellung einer pharmazeutischen Zubereitung, enthaltend (S)-3-(2-((S)-4-(4-(Amino-imino-methyl)-phenyl)-4-methyl-2,5-dioxo-imidazolidin-1-yl)-acetylamino)-3-phenyl-propionsäure-ethylester-Hydrogenmaleat nach Anspruch 1 und/oder 2 und/oder ein oder mehrere physiologisch verträgliche Salze davon als Wirkstoff, **dadurch gekennzeichnet, daß** man diese zusammen mit pharmazeutisch annehmbaren Träger- und Zusatzstoffen und gegebenenfalls noch einem oder mehreren anderen pharmakologischen Wirkstoffen in eine geeignete Darreichungsform bringt.

## Claims

1. Ethyl 3-(2-(4-(4-(aminoiminomethyl)phenyl)-4-methyl-2,5-dioxoimidazolidin-1-yl)acetylamino)-3-phenylpropionate hydrogen maleate of the formula I, 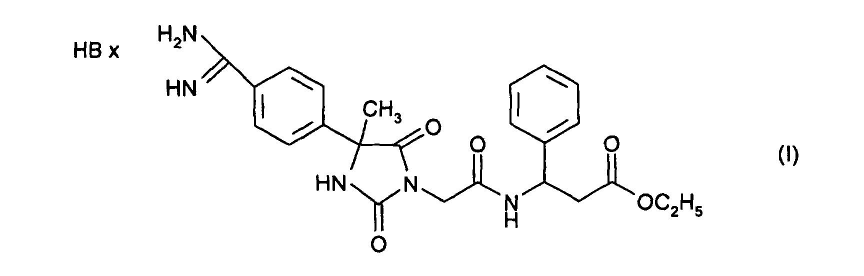 in which HB is maleic acid and the (S) configuration is present at the chiral center in the imidazolidine ring and the (S) configuration is present at the chiral center in the propionic acid unit, and the physiologically tolerated salts thereof.

2. Ethyl (S)-3-(2-((S)-4-(4-(aminoiminomethyl)phenyl)-4-methyl-2,5-dioxoimidazolidin-1-yl)acetylamino)-3-phenylproprionate hydrogen maleate.

3. A process for preparing ethyl (S)-3-(2-((S)-4-(4-(aminoiminomethyl)phenyl)-4-methyl-2,5-dioxoimidazolidin-1-yl)acetylamino)-3-phenylpropionate hydrogen maleate as claimed in claim 1 or 2, which comprises carrying out an anion exchange with maleic acid and/or maleates using compounds of the formula II, 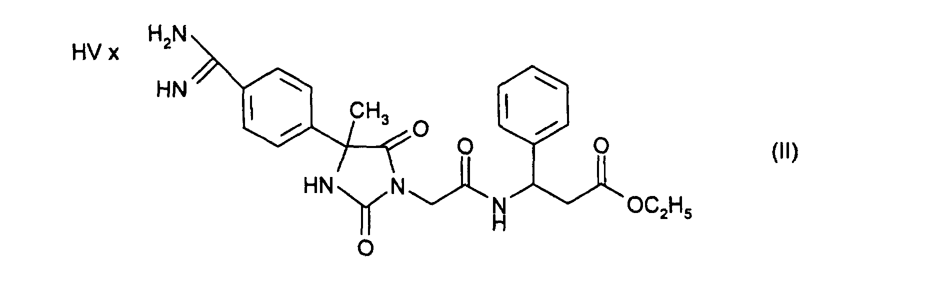 in which HV is an arbitrary inorganic or organic acid which is different from maleic acid and the (S) configuration is present at the chiral center in the imidazolidine ring and the (S) configuration is present at the chiral center in the propionic acid unit.

4. The process as claimed in claim 3, wherein, for the anion exchange, compounds of the formula II are chromatographed through an ion exchange material which is loaded with maleic acid.

5. The process as claimed in claim 3, wherein, for the anion exchange, compounds of the formula II are brought into contact with maleic acid or maleates in a solvent.

6. The process as claimed in one or more of claims 3 to 5, wherein the anion exchange is carried out using the acetic acid salt of the formula IIb 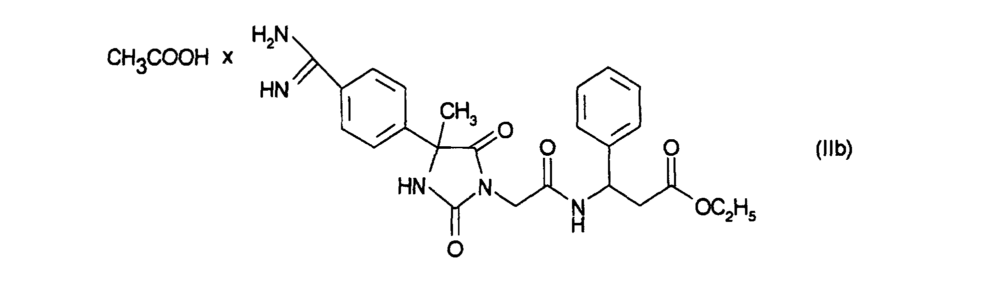 in which the (S) configuration is present at the chiral center in the imidazolidine ring and the (S) configuration is present at the chiral center in the propionic acid unit.

7. The process as claimed in one or more of claims 3 to 6, wherein, for preparing the compounds of the formula II, the compound of the formula III 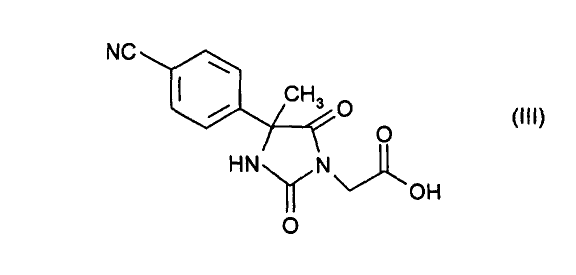 and the compound of the formula IV 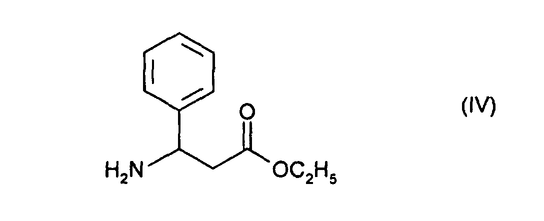 are coupled to give the compound of the formula V, 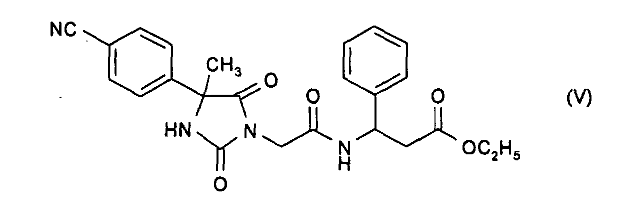 the latter is converted with hydroxylamine into the compound of the formula VI, 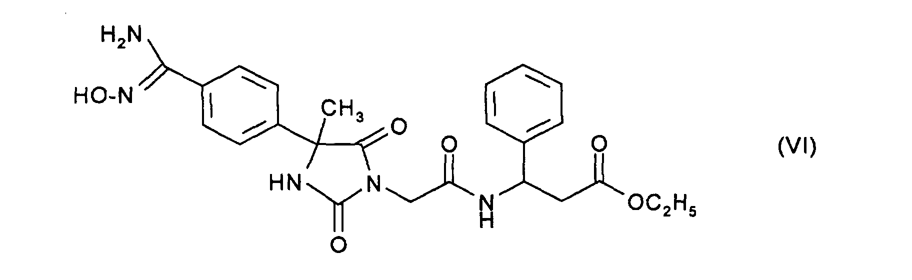 and the latter is converted, by hydrogenation and addition of the acid of the formula HV, into the compounds of the formula II, preferably by hydrogenation in the presence of acetic acid into the compound of the formula IIb, where, in the compounds of the formula III, IV, V and VI, the (S) configuration is present at the chiral center in the imidazolidine ring and the (S) configuration is present at the chiral center in the propionic acid unit.

8. Ethyl (S)-3-(2-((S)-4-(4-(aminoiminomethyl)phenyl)-4-methyl-2,5-dioxoimidazolidin-1-yl)acetylamino)-3-phenylpropionate hydrogen maleate as claimed in claim 1 and/or 2 and/or a physiologically tolerated salt thereof for use as pharmaceuticals.

9. Ethyl (S)-3-(2-((S)-4-(4-(aminoiminomethyl)phenyl)-4-methyl-2,5-dioxoimidazolidin-1-yl)acetylamino)-3-phenylpropionate hydrogen maleate as claimed in claim 1 and/or 2 and/or a physiologically tolerated salt thereof for use as inhibitors of thrombocyte aggregation, for use in the prevention of thromboses or for use as inhibitors of the metastasis of carcinoma cells.

10. The use of ethyl (S)-3-(2-((S)-4-(4-(aminoiminomethyl)phenyl)-4-methyl-2,5-dioxoimidazolidin-1-yl)acetylamino)-3-phenylpropionate hydrogen maleate as claimed in claim 1 and/or 2, and/or its physiologically tolerated salts, for preparing pharmaceuticals for inhibiting thrombocyte aggregation, for preventing thromboses or for inhibiting the metastasis of carcinoma cells.

11. Ethyl (S)-3-(2-((S)-4-(4-(aminoiminomethyl)phenyl)-4-methyl-2,5-dioxoimidazolidin-1-yl)acetylamino)-3-phenylpropionate hydrogen maleate as claimed in claim 1 and/or 2 and/or a physiologically tolerated salt thereof for treating or preventing diseases of the coronary vascular system or the cerebrovascular system, peripheral arterial diseases or venous or microcirculatory vascular diseases, or for use in association with dialysis or extracorporeal circulation.

12. The use of ethyl (S)-3-(2-((S)-4-(4-(aminoiminomethyl)phenyl)-4-methyl-2,5-dioxoimidazolidin-1-yl)acetylamino)-3-phenylpropionate hydrogen maleate as claimed in claim 1 and/or 2, and/or its physiologically tolerated salts, for preparing pharmaceuticals for treating or preventing diseases of the coronary vascular system or the cerebrovascular system, peripheral arterial diseases or venous or microcirculatory vascular diseases, or pharmaceuticals which are employed in association with dialysis or extracorporeal circulation.

13. A pharmaceutical preparation, which comprises ethyl (S)-3-(2-((S)-4-(4-(aminoiminomethyl)phenyl)-4-methyl-2,5-dioxoimidazolidin-1-yl)acetylamino)-3-phenylpropionate hydrogen maleate as claimed in claim 1 and/or 2, and/or one or more physiologically tolerated salts thereof, as the active compound together with pharmaceutically acceptable carrier substances and additives and, optionally, one or more different pharmacological active compounds in addition.

14. A process for producing a pharmaceutical preparation comprising ethyl (S)-3-(2-((S)-4-(4-(aminoiminomethyl)-phenyl)-4-methyl-2,5-dioxoimidazolidin-1-yl)acetylamino)-3-phenylpropionate hydrogen maleate as claimed in claim 1 and/or 2, and/or one or more physiologically tolerated salts thereof, as the active compound, which comprises bringing the latter, together with pharmaceutically acceptable carrier substances and additives and, optionally, one or more different pharmacological active compounds in addition, into a suitable form for administration.

## Revendications

1. Hydrogénomaléate de l'ester éthylique de l'acide 3-(2-(4-(4-(aminoiminométhyl)phényl)-4-méthyl-2,5-dioxoimidazolidin-1-yl)-acétylamino)-3-phénylpropionique de formule I, 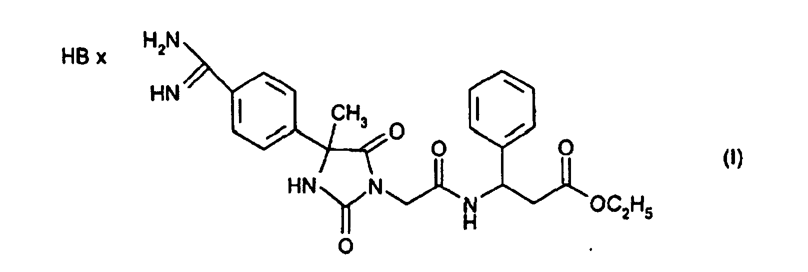 dans laquelle HB signifie l'acide maléique et le centre chiral dans le cycle imidazolidine présente la configuration (S) et le centre chiral dans l'unité acide propionique présente la configuration (S), et ses sels physiologiquement acceptables.

2. Hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique.

3. Procédé pour la préparation de l'hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique selon la revendication 1 ou 2, **caractérisé en ce qu'**on réalise un échange anionique avec de l'acide maléique et/ou des maléates avec des composés de formule générale II, 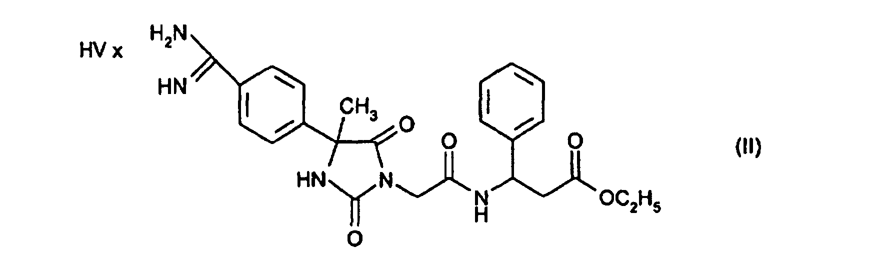 dans laquelle HV représente un acide inorganique ou organique quelconque, différent de l'acide maléique, et le centre chiral dans le cycle imidazolidine présente la configuration (S) et le centre chiral dans l'unité acide propionique présente la configuration (S).

4. Procédé selon la revendication 3, **caractérisé en ce que**, pour l'échange anionique, les composés de formule générale II sont soumis à une chromatographie sur un échangeur d'ions chargé d'acide maléique.

5. Procédé selon la revendication 3, **caractérisé en ce que**, pour l'échange anionique, on rassemble des composés de formule générale II dans un solvant avec de l'acide maléique ou des maléates.

6. Procédé selon l'une ou plusieurs des revendications 3 à 5, **caractérisé en ce que** l'échange d'anions est réalisé avec le sel d'acide acétique de formule IIb, 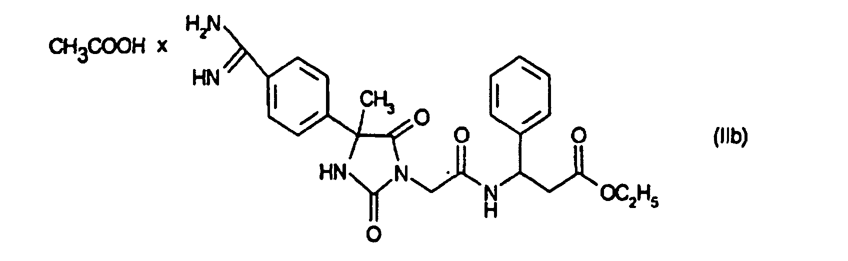 dans laquelle le centre chiral dans le cycle imidazolidine présente la configuration (S) et le centre chiral dans l'unité acide propionique présente la configuration (S).

7. Procédé selon l'une ou plusieurs des revendications 3 à 6, **caractérisé en ce que** pour la préparation des composés de formule générale II, le composé de formule III 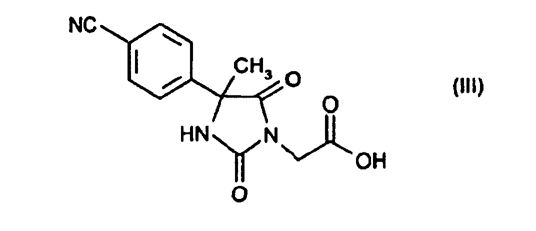 et le composé de formule IV, 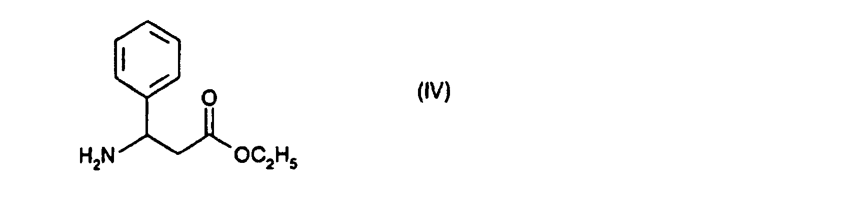 sont couplés en composé de formule V 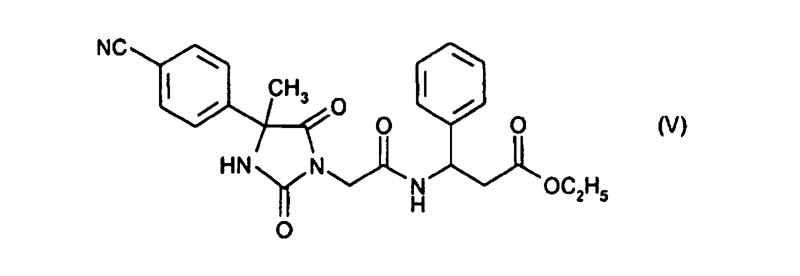 celui-ci est transformé avec de l'hydroxylamine en composé de formule VI 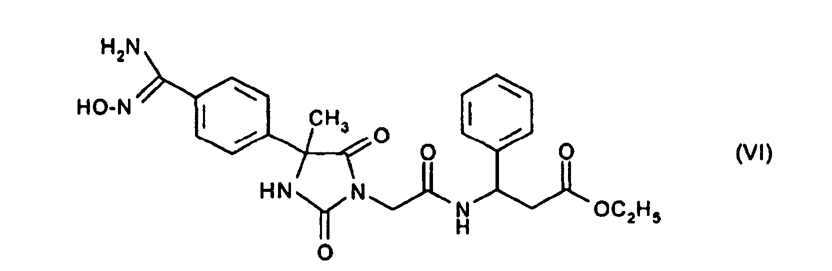 et celui-ci est transformé par hydrogénation et addition de l'acide de formule générale HV en composés de formule générale II, de préférence par hydrogénation en présence d'acide acétique en composé de formule IIb, où, dans les composés de formules III, IV, V et VI, le centre chiral dans le cycle imidazolidine présente la configuration (S) et le centre chiral dans l'unité acide propionique présente la configuration (S).

8. Hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique selon la revendication 1 et/ou 2 et/ou ses sels physiologiquement acceptables destinés à une utilisation comme médicament.

9. Hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique selon la revendication 1 et/ou 2 et/ou ses sels physiologiquement acceptables destinés à une utilisation comme inhibiteurs de l'agrégation de thrombocytes, à une utilisation pour empêcher les thromboses ou à une utilisation comme inhibiteurs de la formation de métastases de cellules cancéreuses.

10. Utilisation d'hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique selon la revendication 1 et/ou 2 et/ou ses sels physiologiquement acceptables pour la préparation de médicaments destinés à inhiber l'agrégation de thrombocytes, à empêcher les thromboses ou à inhiber la formation de métastases de cellules cancéreuses.

11. Hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique selon la revendication 1 et/ou 2 et/ou ses sels physiologiquement acceptables pour le traitement ou la prévention de maladies du système coronaro-vasculaire ou cérébro-vasculaire, de maladies artérielles périphériques ou de maladies vasculaires veineuses ou microcirculatoires ou pour l'utilisation lors de la dialyse ou de la circulation extra-corporelle.

12. Utilisation de l'hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique selon la revendication 1 et/ou 2 et/ou de ses sels physiologiquement acceptables pour la préparation de médicaments destinés au traitement ou à la prévention de maladies du système coronaro-vasculaire ou cérébro-vasculaire, de maladies artérielles périphériques ou de maladies vasculaires veineuses ou microcirculatoires ou de médicaments utilisés lors de la dialyse ou de la circulation extra-corporelle.

13. Composition pharmaceutique, **caractérisée en ce qu'**elle contient l'hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique selon la revendication 1 et/ou 2 et/ou un ou plusieurs sels physiologiquement acceptables de celui-ci comme substance active avec des substances support ou des additifs pharmaceutiquement acceptables et le cas échéant encore une ou plusieurs autres substances actives pharmacologiques.

14. Procédé pour la préparation d'une composition pharmaceutique, contenant l'hydrogénomaléate de l'ester éthylique de l'acide (S)-3-(2-((S)-4-(4-(aminoiminométhyl)phényl)-4-méthyl-2,5-dioxoimidazolidin-1-yl)-acétylamino)-3-phénylpropionique selon la revendication 1 et/ou 2 et/ou un ou plusieurs sels physiologiquement acceptables de celui-ci comme substance active, **caractérisé en ce qu'**on amène ceux-ci dans une forme d'administration appropriée avec des substances support et des additifs pharmaceutiquement acceptables et le cas échéant encore une ou plusieurs autres substances actives pharmacologiques.
